# EUROPEAN PATENT SPECIFICATION

(11) **EP 3 090 049 B1**
(45) Date of publication and mention of the grant of the patent: **18.10.2017**
(21) Application number: 14821164.2
(22) Date of filing: 19.12.2014
(51) Int. Cl.: C12N 15/09

(54) **ENRICHMENT OF FULL-LENGTH OLIGONUCLEOTIDES VIA TRANSCRIPTION/TRANSLATION-MEDIATED PURIFICATION**
ANREICHERUNG VOLLSTÄNDIGER OLIGONUKLEOTIDE ÜBER TRANSKRIPTIONS-/TRANSLATIONSVERMITTELTE REINIGUNG
ENRICHISSEMENT D'OLIGONUCLEOTIDES DE PLEINE LONGUEUR PAR TRANSCRIPTION/PURIFICATION À MÉDIATION PAR TRANSLATION

(30) Priority: 31.12.2013 US 201361922267 P
(43) Date of publication of application: 09.11.2016
(73) Proprietor: F. Hoffmann-La Roche AG, 4070 Basel (CH); Roche Diagnostics GmbH, 68305 Mannheim (DE)
(72) Inventor: PATEL, Jigar, Verona, WI 53593 (US); LYAMICHEV, Victor, Madison, Wisconsin 53711 (US); LO, Ken, Madison, WI 53719 (US); GOODRICH, Lauren, Madison, Wisconsin 53715 (US)
(74) Representative: Burger, Alexander
(86) International application number: PCT/EP2014/078662
(87) International publication number: WO 2015/101516

(56) References cited:
- WO-A1-01/07657
- BARENDT ET AL: "Streamlined Protocol for mRNA Display", ACS COMB. SCI, vol. 15, 10 January 2013 (2013-01-10), pages 77-81, XP002736718,
- ZHOU MING-YI ET AL: "Universal TA cloning", CURRENT INNOVATIONS IN MOLECULAR BIOLOGY SERIES; GENE CLONING AND ANALYSIS: CURRENT INNOVATIONS HORIZON PRESS INC. {A}, 156 FIFTH AVE., NEW YORK, NEW YORK 10010, USA SERIES : CURRENT INNOVATIONS IN MOLECULAR BIOLOGY SERIES, 2 January 2000 (2000-01-02), pages 1-7, XP002736741,
- MARSISCHKY GERALD ET AL: "Many paths to many clones: A comparative look at high-throughput cloning methods", GENOME RESEARCH, vol. 14, no. 10B, October 2004 (2004-10), pages 2020-2028, XP002736742, ISSN: 1088-9051
- SAGERSTROM C G ET AL: "Subtractive cloning: Past, present, and future", ANNUAL REVIEW OF BIOCHEMISTRY ANNUAL REVIEWS INC. {A}, P.O. BOX 10139, 4139 EL CAMINO WAY, PALO ALTO, CALIFORNIA 94306, USA SERIES : ANNUAL REVIEW OF BIOCHEMISTRY (ISSN 0066-4154), 1997, pages 751-783, XP002736743,

### SEQUENCE LISTING

<110> Roche Diagnostics GmbH F. Hoffmann-La Roche AG
<120> ENRICHMENT OF FULL-LENGTH OLIGONUCLEOTIDES VIA TRANSCRIPTION/TRANSLATION-MEDIATED PURIFICATION
<130> P31918-WO
<140>
   <141>
<150> 61/922,267
   <151> 2013-12-31
<160> 18
<170> PatentIn version 3.5
<210> 1
   <211> 15
   <212> DNA
   <213> Artificial Sequence
<220>
   <221> source
   <223> /note="Description of Artificial Sequence: Synthetic oligonucleotide"
<400> 1
   tgccggagtc agcgt 15
<210> 2
   <211> 15
   <212> DNA
   <213> Artificial Sequence
<220>
   <221> source
   <223> /note="Description of Artificial Sequence: Synthetic oligonucleotide"
<400> 2
   agtcagagtc gccac 15
<210> 3
   <211> 64
   <212> DNA
   <213> Artificial Sequence
<220>
   <221> source
   <223> /note="Description of Artificial Sequence: Synthetic oligonucleotide"
<400> 3
   taatacgact cactataggg ttaactttag taaggaggac agctaaatgt gccggagtca 60
   gcgt 64
<210> 4
   <211> 65
   <212> DNA
   <213> Artificial Sequence
<220>
   <221> source
   <223> /note="Description of Artificial Sequence: Synthetic oligonucleotide"
<400> 4
   taatacgact cactataggg ttaactttag taaggaggac agctaaatgc tgccggagtc 60
   agcgt 65
<210> 5
   <211> 66
   <212> DNA
   <213> Artificial Sequence
<220>
   <221> source
   <223> /note="Description of Artificial Sequence: Synthetic oligonucleotide"
<400> 5
   taatacgact cactataggg ttaactttag taaggaggac agctaaatgc ctgccggagt 60
   cagcgt 66
<210> 6
   <211> 70
   <212> DNA
   <213> Artificial Sequence
<220>
   <221> source
   <223> /note="Description of Artificial Sequence: Synthetic oligonucleotide"
<400> 6
   tttttttttt tcaaactgcg gatggctcca acttatcgtc gtcatctttg tagtcagtca 60
   gagtcgccac 70
<210> 7
   <211> 45
   <212> DNA
   <213> Artificial Sequence
<220>
   <221> source
   <223> /note="Description of Artificial Sequence: Synthetic oligonucleotide"
<400> 7
   ttttttctta tcgtcgtcat ctttgtagtc agtcagagtc gccac 45
<210> 8
   <211> 49
   <212> DNA
   <213> Artificial Sequence
<220>
   <221> source
   <223> /note="Description of Artificial Sequence: Synthetic oligonucleotide"
<400> 8
   taatacgact cactataggg ttaactttag taaggaggac agctaaatg 49
<210> 9
   <211> 55
   <212> DNA
   <213> Artificial Sequence
<220>
   <221> source
   <223> /note="Description of Artificial Sequence: Synthetic oligonucleotide"
<400> 9
   tttttttttt tcaaactgcg gatggctcca acttatcgtc gtcatctttg tagtc 55
<210> 10
   <211> 30
   <212> DNA
   <213> Artificial Sequence
<220>
   <221> source
   <223> /note="Description of Artificial Sequence: Synthetic oligonucleotide"
<400> 10
   ttttttctta tcgtcgtcat ctttgtagtc 30
<210> 11
   <211> 8
   <212> PRT
   <213> Artificial Sequence
<220>
   <221> source
   <223> /note="Description of Artificial Sequence: Synthetic peptide"
<400> 11
   Asp Tyr Lys Asp Asp Asp Asp Lys
   1               5
<210> 12
   <211> 8
   <212> PRT
   <213> Artificial Sequence
<220>
   <221> source
   <223> /note="Description of Artificial Sequence: Synthetic peptide"
<400> 12
   Trp Ser His Pro Gln Phe Glu Lys
   1               5
<210> 13
   <211> 169
   <212> DNA
   <213> Artificial Sequence
<220>
   <221> source
   <223> /note="Description of Artificial Sequence: Synthetic polynucleotide"
<400> 13
   taatacgact cactataggg ttaactttag taaggaggac agctaaatgt gccggagtca      60
   gcgtcctcat cctcatggaa tctccacttg gtgcggggat agacatttga tgcagggaga     120
   acaggtggcg actctgactg actacaaaga tgacgacgat aagaaaaaa                 169
<210> 14
   <211> 192
   <212> DNA
   <213> Artificial Sequence
<220>
   <221> source
   <223> /note="Description of Artificial Sequence: Synthetic polynucleotide"
<400> 14
   taatacgact cactataggg ttaactttag taaggaggac agctaaatgt gccggagtca      60
   gcgtcctcat cctcatggaa tctccacttg gtgcggggat agacatttga tgcagggaga     120
   acaggtggcg actctgactg actacaaaga tgacgacgat aagttggagc catccgcatt     180
   tgaaaaaaaa aa                                                         192
<210> 15
   <211> 48
   <212> PRT
   <213> Artificial Sequence
<220>
   <221> source
   <223> /note="Description of Artificial Sequence: Synthetic polypeptide"
<400> 15
   Met Cys Arg Ser Gln Arg Pro His Pro His Gly Ile Ser Thr Trp Cys
   1               5                   10                  15
   Gly Asp Arg His Leu Met Gln Gly Glu Gln Val Ala Thr Leu Thr Asp
               20                  25                  30
   Tyr Lys Asp Asp Asp Asp Lys Leu Glu Pro Ser Ala Val Lys Lys Lys
           35                  40                  45
<210> 16
   <211> 24
   <212> DNA
   <213> Artificial Sequence
<220>
   <221> source
   <223> /note="Description of Artificial Sequence: Synthetic oligonucleotide"
<400> 16
   tgccggagtc agcgtcctca tcct 24
<210> 17
   <211> 90
   <212> DNA
   <213> Artificial Sequence
<220>
   <221> source
   <223> /note="Description of Artificial Sequence: Synthetic oligonucleotide"
<400> 17
   tgccggagtc agcgtcctca tcctcatgga atctccactt ggtgcgggga tagacatttg 60
   atgcagggag aacaggtggc gactctgact 90
<210> 18
   <211> 24
   <212> DNA
   <213> Artificial Sequence
<220>
   <221> source
   <223> /note="Description of Artificial Sequence: Synthetic oligonucleotide"
<400> 18
   ggagaacagg tggcgactct gact 24

## Description

### BACKGROUND OF THE INVENTION

In synthesizing oligonucleotide of length N, by-products of length N+/-1, N+/-2, etc., exist in abundance. This inherent error is directly related to the fidelity of the synthesis process. For applications where the incomplete products do not interfere with the result, oligonucleotides can be used as is. However, for fidelity-demanding applications, such as gene and genome assembly, purification is necessary to ensure the assembled genes or genomes are nearly error-free. Traditional oligonucleotide purification methods such as reverse phase and anion-exchange HPLC can separate full length (i.e., desired length) products from its N+/-1 and N+/-2 by-products, but efficiency decreases with increasing oligonucleotide length. The proposed method does not have the same constraints as traditional purification techniques.

The present invention is a method used for selection of oligonucleotides useful in particular applications developed by using certain advantageous properties of mRNA display. Briefly, mRNA display is a technique used for in vitro peptide synthesis and selection to create peptides or proteins that can bind to a desired target with high affinity or selectivity. The process results in translated peptides or proteins that are linked to their mRNA progenitor via a puromycin linkage. The complex then binds to an immobilized target in a selection step (affinity chromatography). The mRNA-protein fusions that bind well can then be reverse transcribed to cDNA and their sequence amplified via polymerase chain reaction. The result is a nucleotide sequence that encodes a peptide with high affinity for the molecule of interest.

The present invention uses certain aspects of mRNA display to select for or purify oligonucleotides that have the correct nucleotide sequence. The present invention takes advantage of the fact that a slight change in nucleotide sequence (addition, deletion, substitution, etc.) can cause large scale changes in the peptide expressed by the nucleotide sequence.

### SUMMARY OF THE INVENTION

In one embodiment, the invention is a method of purifying a full-length target oligonucleotide from a pool further containing shorter or longer same-sequence oligonucleotide by-products, the method comprising: amplifying the pool to form a second pool of oligonucleotides; transcribing the second pool of oligonucleotides to form an RNA pool wherein the RNA molecules contain codons for at least one tag sequence; ligating the molecules of the RNA pool to puromycin; translating the puromycin-ligated RNA molecules to form a pool of chimeric molecules containing the RNA linked to a peptide expressed from the RNA; capturing and isolating the chimeric molecules that express the tag; reverse transcribing the RNA of the isolated chimeric moieties to form a pool of cDNA comprising the purified full-length oligonucleotide. Is some embodiments, the amplification uses at least two primers comprising a target binding site, and further comprising one or more of a promoter, an enhancer, a ribosome binding site, a translation initiation site and a sequence encoding at least one tag. In some embodiments, the first primer comprises promoter, enhancer, the ribosome binding site and the translation initiation site and the second primer comprises the sequence encoding at least one tag. In some embodiments, the amplification uses the second primer comprising the sequence encoding a first tag and a sequence encoding a second tag. In some embodiments, the second primer further comprises a sequence encoding a third tag. In some embodiments, the first primer comprises a target specific sequence conjugated to SEQ ID NO: 8. For example, the first primer comprises SEQ ID NO: 3. In some embodiments, the second primer comprises a target specific sequence conjugated to SEQ ID NOs: 9 or 10. For example, the second primer comprises SEQ ID NO: 6 or 7. In some embodiments the codons of the first tag sequence are in frame with the full-length oligonucleotide and out of frame with shorter and with longer same-sequence oligonucleotide by-products. In other embodiments, the codons of the second tag sequence are in frame with - 1 shorter and with + 2 longer same-sequence oligonucleotide by-products and out of frame with the full-length oligonucleotide. In other embodiments, the codons of the third tag sequence are in frame with - 2 shorter and with + 1 longer same-sequence oligonucleotide by-products and out of frame with the full-length oligonucleotide. In some embodiments the capturing is performed with a tag-specific binding agent, for example, an antibody. In some embodiments, capturing comprises capturing of the first tag sequence. In some embodiments, capturing comprises capturing of the second tag sequence. In some embodiments, capturing comprises capturing of the third tag sequence. In some embodiments, the method further comprises amplifying the pool of cDNA comprising the purified full-length oligonucleotide. In some embodiments, the method further comprises repeating one or more cycles of steps of amplifying, transcribing, ligating the molecules to puromycin, translating, capturing and isolating, and reverse transcribing using the amplified pool of cDNA as the second pool of oligonucleotides.

In other embodiments, the invention is a kit for purifying a full-length target oligonucleotide from a pool further containing shorter or longer same-sequence oligonucleotide by-products, the method comprising a pair of primers wherein the first primer comprises a target binding site, a promoter, an enhancer, a ribosome binding site and a translation initiation site and the second primer comprises the sequence encoding at least one tag. In some embodiments, the kit further comprises one or more of the following: reagents for DNA amplification, reagents for DNA transcription, reagents for nucleic acid ligation, puromycin, reagents for RNA translation, at least one tag-binding agent specific for the at least one tag and reagents for RNA reverse transcription. In some embodiments, the second primer comprises the sequence encoding a first tag; and further comprises a sequence encoding a second tag. In some embodiments, the second primer further comprises a sequence encoding a third tag. In some embodiments, the first primer comprises a target specific sequence conjugated to SEQ ID NO: 8 and the second primer comprises a target specific sequence conjugated to SEQ ID NOs: 9 or 10. In some embodiments, the first primer comprises SEQ ID NO: 3 and the second primer comprises SEQ ID NOs: 6 or 7.

### BRIEF DESCRIPTION OF THE FIGURES

**FIGURE 1** is an example of an oligonucleotide (SEQ ID NO: 13) with appropriate promoter elements and a tag, in this case FLAG(SEQ ID Nos: 11), to indicate in-phase transcription.
**FIGURE 2** is a schematic demonstrating an embodiment of the present invention.
**FIGURE 3** is a diagram illustrating the two-tag system. The oligonucleotide is disclosed as SEQ ID NO: 14 while the two tags (FLAG and Strep) are disclosed as SEQ ID NOS 11-12, respectively. The peptide sequence is disclosed as SEQ ID NO: 15.
**FIGURE 4** is a diagram showing the interaction of the primers specific to the target sequence The figure discloses SEQ ID NOS 3, 16, 17, 18, 6, 11, 12, 6, 7, 3, 4, and 5, respectively from top to bottom.
**FIGURE 5** is a gel showing the products of the first step of the inventive method.
**FIGURE 6** is a gel showing the products at various amplification stages of step 7 of the inventive method.
**FIGURE 7** is a graph illustrating the effects of mRNA display purification on synthesized probe length.
**FIGURE 8** is a graph illustrating the amounts of out-of-phase moieties in the original oligonucleotide pool as compared to a pool that has been purified by the present inventive method.

### DETAILED DESCRIPTION OF THE INVENTION

The present invention provides a method of purifying a full-length target oligonucleotide from a pool further containing shorter or longer same-sequence oligonucleotide by-products, the method comprising:
a) amplifying the pool to form a second pool of oligonucleotides;
b) transcribing the second pool of oligonucleotides to form an RNA pool wherein the RNA molecules contain codons for at least one tag sequence;
c) ligating the molecules of the RNA pool to puromycin;
d) translating the puromycin-ligated RNA molecules to form a pool of chimeric molecules containing the RNA linked to a peptide expressed from the RNA;
e) capturing and isolating the chimeric molecules that express the tag;
f) reverse transcribing the RNA of the isolated chimeric moieties to form a pool of cDNA comprising the purified full-length oligonucleotide.

The amplification in step a) may use at least two primers comprising a target binding site, and further comprising one or more of a promoter, an enhancer, a ribosome binding site, a translation initiation site and a sequence encoding at least one tag.

The first primer may comprise a promoter, an enhancer, the ribosome binding site and the translation initiation site and the second primer may comprise the sequence encoding at least one tag. The amplification in step a) may further use a second primer comprising the sequence encoding a first tag and a sequence encoding a second tag. Then, the codons of the second tag sequence may be in frame with - 1 shorter and with + 2 longer same-sequence oligonucleotide by-products and out of frame with the full-length oligonucleotide. In this case, the capturing in step e) may comprise capturing of the second tag sequence. The amplification in step a) may use a second primer that further comprises a sequence encoding a third tag. Then, the codons of the third tag sequence may be in frame with a - 2 shorter and with a + 1 longer same-sequence oligonucleotide by-products and out of frame with the full-length oligonucleotide. 17. The capturing in step e) may comprise capturing of the third tag sequence

The first primer may comprises a target specific sequence conjugated to SEQ ID NO: 8. For example, the first primer may comprise SEQ ID NO: 3. The second primer may comprise a target specific sequence conjugated to SEQ ID NOs: 9 or 10. For example, the second primer may comprise SEQ ID NO: 6 or 7.

The codons of the first tag sequence may be in frame with the full-length oligonucleotide and out of frame with shorter and with longer same-sequence oligonucleotide by-products. 15. Then, the capturing in step e) may comprise capturing of the first tag sequence.

The capturing in step e) may be performed with a tag-specific binding agent, which may be an antibody. The amplifying in step a) may result in amplification of purified full-length oligonucleotide.

In particular embodiment, one or more cycles of steps b)-f) are repeted using the amplified pool of cDNA as the second pool of oligonucleotides.

The present invention also provides a kit for purifying a full-length target oligonucleotide from a pool further containing shorter or longer same-sequence oligonucleotide by-products, the method comprising a pair of primers wherein the first primer comprises a target binding site, a promoter, an enhancer, a ribosome binding site and a translation initiation site and the second primer comprises the sequence encoding at least one tag. The kit may further comprise one or more of the following: reagents for DNA amplification, reagents for DNA transcription, reagents for nucleic acid ligation, puromycin, reagents for RNA translation, at least one tag-binding agent specific for the at least one tag and reagents for RNA reverse transcription. The second primer may comprise the sequence encoding a first tag; and the further a sequence encoding a second tag. The second primer may further comprise a sequence encoding a third tag. In one embodiment, the first primer comprises a target specific sequence conjugated to SEQ ID NO: 8 and the second primer comprises a target specific sequence conjugated to SEQ ID NOs: 9 or 10. In another embodiment, the first primer comprises SEQ ID NO: 3 and the second primer comprises SEQ ID NOs: 6 or 7.

### DEFINITIONS

The term "amplification" refers to the production of a plurality of nucleic acid molecules from a target nucleic acid. Amplification can be carried out by any method generally known in the art, such PCR, RT-PCR, Isothermal Amplification, Ligase Chain Reaction (LCR), Polymerase Ligase Chain Reaction, Gap-LCR, Repair Chain Reaction, 3SR, NASBA, Strand Displacement Amplification (SDA), Transcription Mediated Amplification (TMA), and Qb-amplification. In some amplification methods, primers hybridize to specific sites on the target nucleic acid molecules in order to provide an initiation site for extension by a polymerase.

The term "complementary" refers to the ability to form favourable thermodynamic stability and specific pairing between the bases of two nucleotides in a nucleic acid at an appropriate temperature and ionic buffer conditions. This pairing is dependent in part, on the hydrogen bonding properties of each nucleotide. Oligonucleotides, e.g., primers for amplification of target nucleic acids can be both fully complementary over their entire length with a target nucleic acid molecule or "partially complementary" wherein the primer contains some bases non-complementary to the corresponding base in a target nucleic acid.

The term "detecting" means assessing the presence or absence of a target nucleic acid in a sample.

The term "enriched" refers to any method of treating a sample comprising a target nucleic acid that allows one to separate the target nucleic acid from at least a part of other material present in the sample. "Enrichment" can thus be understood as a production of a higher relative amount of target nucleic acid over other material. The terms "purify" or "purified" can be used interchangeably with the terms "enrich" or "enriched."

The term "excess" refers to a larger quantity or concentration of a certain reagent as compared to another reagent.

The term "hybridize" refers to the base-pairing between different nucleic acid molecules consistent with their nucleotide sequences. The terms "hybridize" and "anneal" can be used interchangeably.

The terms "nucleic acid" or "polynucleotide" can be used interchangeably and refer to a polymer of ribonucleic acid (RNA) or deoxyribonucleic acid (DNA), or an analog thereof, i.e., polymers including one or more synthetic or modified subunits. Exemplary modifications include methylation, substitution of one or more of the naturally occurring nucleotides with an analog, internucleotide modifications such as uncharged linkages (e.g., methyl phosphonates, phosphotriesters, phosphoamidates, carbamates, and the like), pendent moieties (e.g., polypeptides), intercalators (e.g., acridine, psoralen, and the like), chelators, alkylators, and modified linkages (e.g., alpha anomeric nucleic acids and the like). Also included are synthetic molecules that mimic polynucleotides in their ability to bind to a designated sequence via hydrogen bonding and other chemical interactions. Typically, the nucleotide monomers are linked via phosphodiester bonds, although synthetic forms of nucleic acids can comprise other linkages (e.g., peptide nucleic acids as described in Nielsen et al. (Science 254:1497-1500, 1991). A nucleic acid can be single or double-stranded and is not limited to any particular length.

The term "nucleotide" (unless otherwise specified) in addition to referring to the naturally occurring ribonucleotides or deoxyribonucleotides refer to related structural variants thereof, including derivatives and analogs, that are functionally equivalent with respect to the particular context in which the nucleotide is being used (e.g., hybridization to a complementary base), unless the context clearly indicates otherwise.

The term "oligonucleotide" refers to a nucleic acid that includes at least two nucleic acid monomer units (e.g., nucleotides). An oligonucleotide typically includes from about six to about 175 nucleic acid monomer units, more typically from about eight to about 100 nucleic acid monomer units, and still more typically from about 10 to about 50 nucleic acid monomer units (e.g., about 15, about 20, about 25, about 30, about 35, or more nucleic acid monomer units). The exact size of an oligonucleotide will depend on many factors, including the ultimate function or use of the oligonucleotide. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (Meth. Enzymol. 68:90-99, 1979); the phosphodiester method of Brown et al. (Meth. Enzymol. 68:109-151, 1979); the diethylphosphoramidite method of Beaucage et al. (Tetrahedron Lett. 22:1859-1862, 1981); the triester method of Matteucci et al. (J. Am. Chem. Soc. 103:3185-3191, 1981); automated synthesis methods; Maskless Array Synthesis as disclosed in Singh-Gasson et al., Nature Biotechnology, 17: 974-978, 1999, or the solid support method of U.S. Pat. No. 4,458,066, or other methods known to those skilled in the art.

The terms "shorter or longer same-sequence oligonucleotide" or "shorter or longer same-sequence oligonucleotide by-product" are used interchangeably to refer to an oligonucleotide that has the same sequence as the target or desired oligonucleotide except, typically as a result of an imperfect *in vitro* synthesis process, is missing one or more nucleotides or includes additional one or more nucleotides that were erroneously incorporated.

The term "codon" refers to a sequence of three nucleotides in a DNA or RNA molecule that forms a unit of genetic code, i.e., translation of the nucleic acid sequence into an protein sequence.

The term "primer" refers to a polynucleotide capable of acting as a point of initiation of template-directed nucleic acid synthesis when placed under conditions in which polynucleotide extension is initiated (e.g., under conditions comprising the presence of requisite nucleoside triphosphates (as dictated by the template that is copied) and a polymerase in an appropriate buffer and at a suitable temperature or cycle(s) of temperatures (e.g., as in a polymerase chain reaction)). To further illustrate, primers can also be used in a variety of other oligonucleotide-mediated synthesis processes, including as initiators of de novo RNA synthesis and in vitro transcription-related processes (e.g., nucleic acid sequence-based amplification (NASBA), transcription mediated amplification (TMA), etc.). A primer is typically a single-stranded oligonucleotide (e.g., oligodeoxyribonucleotide). The appropriate length of a primer depends on the intended use of the primer but typically ranges from 6 to 40 nucleotides, more typically from 15 to 35 nucleotides. Short primer molecules generally require cooler temperatures to form sufficiently stable hybrid complexes with the template. A primer need not reflect the exact sequence of the template but must be sufficiently complementary to hybridize with a template for primer elongation to occur. In certain embodiments, the term "primer pair" means a set of primers including a 5' sense primer (sometimes called "forward") that hybridizes with the complement of the 5' end of the nucleic acid sequence to be amplified and a 3' antisense primer (sometimes called "reverse") that hybridizes with the 3' end of the sequence to be amplified (e.g., if the target sequence is expressed as RNA or is an RNA). A primer can be labeled, if desired, by incorporating a label detectable by spectroscopic, photochemical, biochemical, immunochemical, or chemical means. For example, useful labels include 32P, fluorescent dyes, electron-dense reagents, enzymes (as commonly used in ELISA assays), biotin, or haptens and proteins for which antisera or monoclonal antibodies are available.

The term "quantifying" as used herein relates to the determination of the amount or concentration of a target nucleic acid present in a sample.

The term "target nucleic acid" is used herein to denote a nucleic acid in a sample which is to be analyzed, i.e. the presence, amount or nucleic acid sequence in a sample is to be determined. The target nucleic acid may be any type of DNA or RNA, a genomic sequence, or a specific gene, or any other fragment thereof.

The term "positive selection" means a purification step wherein the specific binding partner or antibody binds to the desired moiety, allowing the desired moiety to be recovered from a pool containing both the desired and undesired moieties. The term "negative selection" means a purification step wherein the specific binding partner or antibody binds to an undesired moiety and allows its removal, thereby partially or completely purifying the pool of moieties left behind after the removal of the undesired moiety.

The present invention provides a method for separating full length oligonucleotides from oligonucleotides containing errors, such as those that are N+/-1 or N+/-2, etc. To overcome the difficulty in separating full length products, oligonucleotides are translated into their peptide counterparts using mRNA display methods. An RNA-peptide chimera is generated, with the translated peptide covalently linked to its encoded nucleic acid. In the present invention, the mRNA includes nucleotide sequences that encode two specific tags: a tag expressed by in-phase oligonucleotides (called FLAG) and a tag expressed by oligonucleotides that are one step out of phase (called Strep). In some embodiments, no tag is expressed by oligonucleotides that are two steps out of phase. In other embodiments, an additional (third) tag is expressed by oligonucleotides that are two steps out of phase.

When sequences encoding a specific peptide tag are added to the 3' end of the oligonucleotide, only oligonucleotides that are in-frame will translate the "correct" peptide tag (i.e. full length products and rare N+/-3, N+/-6, etc., by-products). During affinity purification of the "correct" peptide tag, followed by amplification of the nucleic acid of the RNA-peptide chimera, enrichment of the desired nucleotide sequences is achieved. Synthesis errors such as N+/-1 and N+/-2 would lead to translation out-of-frame and will translate the "incorrect" peptide tag or no tag. Such synthesis products are removed by the affinity purification.

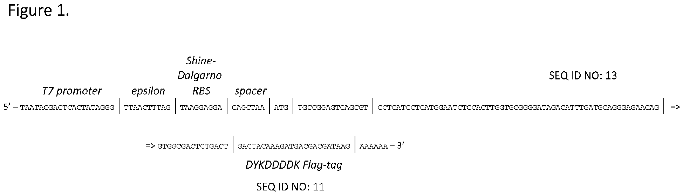

Certain embodiments of the present invention utilize an oligonucleotide that is flanked by amplification sequences on either end. The sequences required for transcription and translation, the sequence encoding the peptide purification tag and an optional poly-A tail are included in the amplification primers. An example of the resulting amplification product is illustrated in Figure 1.

As shown in Figure 1, the oligonucleotide sequence may have several components. One component is a promoter sequence included at or near the 5' end of the nucleotide sequence. In some embodiments, it is the T7 promoter that allows synthesis of RNA using the T7 polymerase in an *in vitro* transcription. Another component may be the epsilon sequence which is a transcription enhancer region located upstream of the start codon. Yet another sequence may be a ribosomal binding site to allow for in vitro translation of the sequence. For example, the Shine-Dalgarno sequence is a ribosomal binding site in prokaryotic mRNA, generally located around 8 bases upstream of the start codon AUG and useful for in vitro translation in a prokaryotic, e.g., E.coli system. For other translation systems, other sequences may be useful. As an example, for translation in a eukaryotic system (e.g., reticulocyte lysate) a deletion mutant of the tobacco mosaic virus (ATMV) 5'UTR is useful.

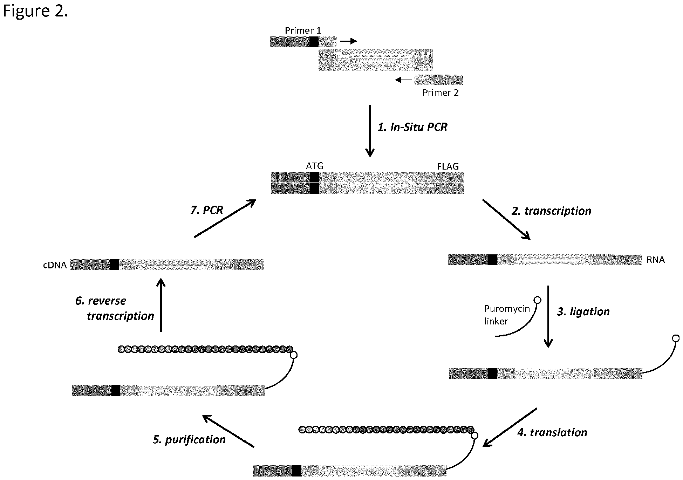

An overview of the present method is found in Figure 2. After PCR, an in vitro transcription reaction (2) converts the amplification products into RNA, which is ligated to an RNA-puromycin linker (3). The resulting RNA-puromycin fusion is translated using an in vitro translation system (4) to yield the RNA-peptide chimera. The RNA in the chimera has two tags the first tag (termed FLAG) is in frame in full-length oligonucleotides and optionally, the second tag (termed Strep) is in frame in oligonucleotides that are one step out of phase. Purification (5) is performed using an anti-FLAG antibody. RNA-peptide chimeras not containing an appropriately translated FLAG tag are washed away during the affinity purification process. A cDNA is generated from the RNA via reverse transcription (6) and amplification (7). The process (steps 2-7) can be repeated to achieve the desired purity.

Looking to the individual steps of Figure 2, in situ PCR (1) is carried out on the targeted sequences using two primers. Primer 1 contains an untranslated region that optionally contains sub-regions such as the promoter region, epsilon sequence, ribosome binding sequence and a spacer region as shown in Figure 1. Downstream from the untranslated region is a start codon (ATG), and attached to the start codon is the target specific region of the primer. Primer 2 also has a target specific region and a coding sequence for one or more tag sequence, in this example, only the FLAG tag coding sequence. The scope of the present invention includes any tag sequences encoding a translation product which is useful for separation according to the present invention, i.e., a peptide product against which an antibody can be generated. The antibody in such a case is then useful for separating those species that have translated the tag sequence from species that have not. Although not shown in Figure 1, Primer 2 can optionally contain a poly-A tail.

Instead of using primers with target-hybridizing regions, Step 1 could also be performed by ligating adaptors on the ends of the desired nucleic acid sequence(s). Such adaptors would contain all the elements that were included in the primers as described above, i.e., the sequences necessary for transcription and translation and tag-coding sequences, as well as the optional poly-A tail. After adaptor ligation, the ligated product could be amplified via PCR (e.g., with primers that specifically hybridize to adaptor sequences) and then subjected to the remaining steps (2-7) of the present method.

After annealing of the primers to the desired nucleic acid sequence, amplification such as in situ PCR is performed, resulting in an amplified, double-stranded PCR product containing the untranslated regions, a start codon, the desired sequence, and a tag-coding region, e.g., FLAG region.

In step 2, after amplification the double-stranded products are transcribed to give the mRNA product. For example, the dsDNA library can be transcribed into mRNA enzymatically using T7 polymerase using methods well known in the art. After the mRNA is generated from the transcription step, the mRNA is conjugated to puromycin, for example, by ligation to an oligonucleotide containing a puromycin at its terminus. The puromycin linker can be formed in any variety of ways. A standard protocol for formation of puromycin oligonucleotides can be found in Liu et al., Meth. Enzym., 318: 268-293 (2000), the disclosure of which is incorporated by reference. Further, puromycin oligonucleotides are commercially available (e.g., Gene Link, Sigma-Aldrich, etc.).

The RNA is ligated to the 3'-puromycin oligonucleotide to form translation templates (see Figure 2, step 3). This ligation step may be accomplished in a variety of ways. For example, translation templates are generated using a splinted ligation of RNA with 5'-phosphorylated puromycin oligonucleotides. Ligation reactions are conducted with RNA, DNA splint, and puromycin oligonucleotides in the presence of a ligase such as T4 DNA ligase. An exemplary method of ligation is found in Liu et al, Meth. Enzym., 318 at 278 (2000). In such methods, the splint should overlap both the 3' end of the RNA and the 5' end of the puromycin oligonucleotide by approximately 10 bases, bringing the ends into alignment. After annealing, the DNA ligase can ligate the RNA to the puromycin oligonucleotides to form the translation templates. However, it is not always necessary to use a DNA splint to achieve ligation. For example, T4 ligase can be used for simple one-to-one ligation without the presence of a DNA splint. While the efficiency of such systems may be less than those that employ the splint DNA, such methods are still sufficient for the present methods. As shown in Figure 2, step 3, the translation templates resulting from the ligation reaction thus contain sequence encoding the untranslated region, the start codon, the target region, the FLAG region, and the puromycin terminus region.

The translation templates are then used in a translation step (see step 4, Figure 2). Translation can be accomplished in a number of different in vitro systems, depending upon the coding used in earlier steps. The most frequently used cell-free translation systems are extracts from rabbit reticulocytes, wheat germ and *Escherichia coli.* The crude extracts containing all the macro components (70S or 80S ribosomes, tRNAs, aminoacyl-tRNA synthetases, initiation, elongation and termination factors, etc.) required for translation of exogenous RNA. To ensure efficient translation, each extract must be supplemented with amino acids, energy sources (ATP, GTP), energy regenerating systems (creatine phosphate and creatine phosphokinase for eukaryotic systems, and phosphoenol pyruvate and pyruvate kinase for the *E. coli* lysate), and other co-factors (Mg²⁺, K⁺, etc.).

*In vitro* translation systems will invariably result in a pool of RNA-peptide chimerae wherein a subpopulation contains the desired nucleotide sequences (resulting in an appropriate expression of the tag), and other subpopulations where the nucleotide sequence is incorrect (e.g., N-1, N-2) resulting in inappropriate expression of the tag.

This mixed pool of chimerae is then purified (see Step 5, Figure 2). The purification step is based upon the presence of the tag (e.g., the FLAG tag) and may utilize an anti-tag antibody to select for those peptides that have appropriately expressed the tag during translation. Several types of purification methods can be used in such embodiments. For example, the purification could be performed by mixing the pool of chimerae with para-magnetic beads conjugated with an anti-tag antibody and separating out the magnetic beads from the sample. Other methods employ the anti-tag antibody bound or conjugated to a solid surface, and the chimera that do not contain the tag sequence are washed away after binding. Further methods employ an anti-tag antibody that is capable of binding to a bead or solid surface, mixing this antibody with the pool of chimera, binding the anti-tag antibody to the bead or solid surface, and washing away the unbound chimera.

Another embodiment of the present invention is a two-tag system for both positive selection of appropriate "correct" sequences and negative selection against inappropriate "incorrect" sequences. As an example, the experimental layout of a two-tag strategy using FLAG tag for positive selection and Strep tag for negative selection is described. It is not necessary in a one- or two-tag system to use FLAG and Strep tags. Other tags against which specific binders can be generated would be useful in this invention as well. In such a system, one tag would be expressed if the sequence remains properly in-frame, whereas a second tag would be expressed if the sequence is out of frame (for example, if the nucleotide sequence being transcribed and translated is N+/-1 or N+/-2). It is noted that the N-1 oligonucleotide will be in the same frame express the same tag as the N+2 oligonucleotide. The N-2 oligonucleotide will be in the same frame express the same tag as the N+1 oligonucleotide. For negative selection, after translation, antibodies raised against the second tag could be used to specifically bind the chimerae where the peptide is out of frame and remove these chimerae from the pool. The purified pool of chimera could then be subjected to an antibody raised against the first tag (positive selection) to bind the desired chimerae and allow for washing away of the rest.

Further, it is possible to employ a three-tag system for even greater purification, wherein the first tag indicates in-frame sequence, the second tag indicates a first out-of-frame sequence (e.g., N-1 or N+2) and a third tag indicates a second out-of-frame sequence (e.g. N-2 or N+1). In such a system, antibodies against the second and third tags can be used to remove the two types of out-of-frame chimerae and an antibody against the first tag can be used for the final purification of the in-frame chimerae.

As is readily apparent, the use of more than one tag may not necessarily mean that the system uses "negative selection." For example, some applications may be designed to positively select for N+/-1 or N+/-2 sequences if it is desired to isolate and purify such sequences.

As is also apparent, the purification step of the present invention can be carried out with antibodies selective for the respective tags; however, the present invention should not be limited to only antibody-based purification. Any binding partner that has a specific binding affinity for the respective tag to the essential exclusion of other tags is potentially useful for the purification step of the inventive method. Examples of such binding partners include, but are not limited to, antibodies, polyclonal antibodies, monoclonal antibodies, antibody fragments, peptides and any other moiety which acts as a specific binder against the respective tag.

After the purification step, the isolated and purified chimera is subjected to reverse transcription to yield a cDNA (see step 6, Figure 2). The cDNA pool is in purified form and is enriched for the targeted nucleotide. Amplification of the resulting cDNA pool, e.g., by PCR increases the amount of the desired nucleic acid (see step 7, figure 2). While the cDNA pool resulting from step 6 is highly enriched in the desired nucleic acid, a user may still desire yet greater purity and repeat steps 2-6 or 2-7. Each cycle should increase the purity of the resulting product.

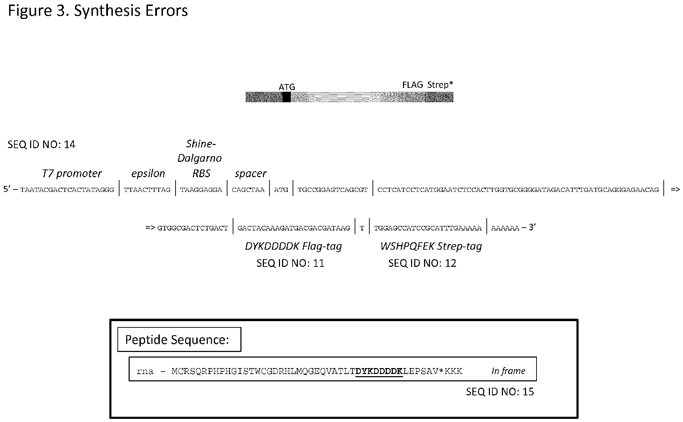

Primer design according to the present invention is illustrated on Figures 3-4. Figure 3 shows a two-tag embodiment of the present invention. Figure 3, top shows the product of the in-situ PCR containing promoters, enhancers, the start codon, the target sequence, and the FLAG tag and Strep tag sequences. Once this oligonucleotide is subjected to mRNA display, mRNA-peptide chimerae are formed as depicted at the bottom of Figure 3. If the nucleotide strand is translated in-frame, the peptide portion of the chimera will contain a properly translated FLAG tag (DYKDDDDK) (SEQ ID NO: 11).. If the nucleotide strand is out-of-frame by one nucleotide (illustrated here as N-1), the peptide portion of the chimera will contain a properly translated Strep tag (WSHPQFEK) (SEQ ID NO: 12).. If the nucleotide strand is out-of-frame by two nucleotides (illustrated here as N-2), neither the FLAG tag nor the Strep tag will be present.

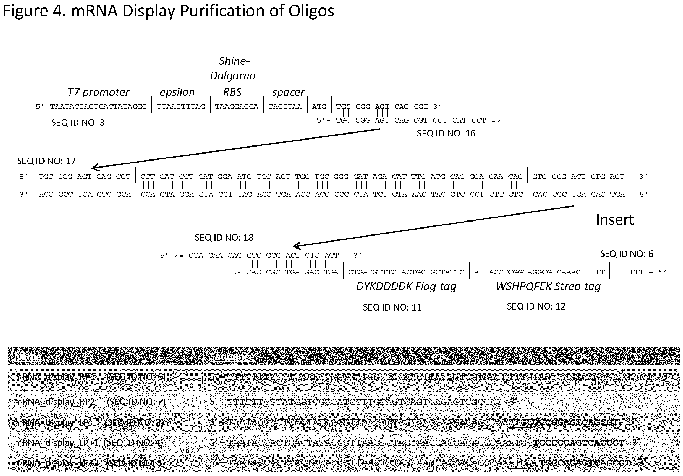

Figure 4 demonstrates how the primers hybridize with the target sequence. The left primer is shown containing a T7 promoter sequence, and epsilon sequence (an enhancer element), a Shine-Dalgarno RBS sequence, and a spacer region adjacent to the target specific region. At the other terminus of the target sequence is depicted the right primer containing both a FLAG tag and a Strep tag.

In some embodiments, primers utilize one or more of the sequences listed in Table 1. The forward primers listed in Table 1 contain the necessary elements such as the promoter sequence, the Shine-Dalgarno sequence, a start codon, and the tags, in this case a FLAG tag and a Strep tag. Segments 1A and 1B are examples of the primer regions specific for the target sequence. It is understood that any other target can be used and a suitable target-specific region of the primer designed therefor. The sequence denoted as MRD_LP is a primer containing a sequence-specific region and a region containing the sequence elements necessary for the steps of the inventive method, i.e., transcription, translation and optionally, reverse transcription. LP is the sequence conjugated to the target-specific sequence.

As shown in Table 1, MRD_RP1 is a primer region used for a two tag method, and includes both a FLAG tag (which would be expressed properly if translation is in-frame) and a Strep tag (which would be expressed properly if translation is one nucleotide out of frame). In this example, the Strep tag is used for negative selection (i.e., those chimerae that have a properly expressed Strep tag are removed from the chimera pool) and the FLAG tag is used for positive selection (i.e., those chimerae that have a properly expressed FLAG tag are the desired in-frame chimerae and are selected). MRD_PR2 is a shorter primer congaing on the FLAG tag. RP1 and RP2 are sequences conjugated to the target-specific sequences.

It is understood that one of skill in the art can utilize the artificial sequences described herein, e.g., LP, RP1 and RP2 in combination with other target-specific sequences in place of sequences 1A and 1B to form forward and reverse primers. It is further understood that the artificial sequences LP, RP1 and RP2 can be modified as long as they contain the essential elements enabling transcription, translation, and reverse transcription as described herein.

**Table 1:**

| SEQ ID NO: | Name | Sequence (5' -3') |
|---|---|---|
| SEQ ID NO: 1 | 1A | TGC CGG AGT CAG CGT |
| SEQ ID NO: 2 | 1B | AGT CAG AGT CGC CAC |
| SEQ ID NO: 3 | MRD LP | |
| SEQ ID NO: 6 | MRD RP1 | |
| SEQ ID NO: 7 | MRD RP2 | |
| SEQ ID NO: 8 | LP | |
| SEQ ID NO: 9 | RP1 | |
| SEQ ID NO: 10 | RP2 | TTT TTT CTT ATC GTC GTC ATC TTT GTA GTC |

### EXAMPLES

### Example 1

### Primer sequences

Primers were designed for purposes of adding the necessary elements such as the promoter sequence, the Shine-Dalgarno sequence, a start codon, and the tags, in this case a FLAG tag and a Strep tag. Table 1 below lists the sequences used in these primers. Segments 1A and 1B show the primer regions specific for the target sequence. The sequence denoted as MRD_LP is a primer region used in primers designed for in-frame (N) translation. To test the method of the invention, out-of-frame sequences were also designed. MRD_LP+1 is a primer region used for detection of translation that is one nucleotide out of frame; MRD_LP+2 is a primer region used for detection of translation that is two nucleotides out of frame. The out-of-frame regions are modified by having an additional cytosine (MRD_LP+1) or two additional cytosines (MRD_LP+2) inserted into the primer sequence after the start codon.

As further seen in Table 2, MRD_RP1 is a primer region used for a two tag method, and includes both a FLAG tag (which would be expressed properly if translation is in-frame) and a Strep tag (which would be expressed properly if translation is one nucleotide out of frame). In this example, the Strep tag is used for negative selection (i.e., those chimerae that have a properly expressed Strep tag are removed from the chimera pool) and the FLAG tag is used for positive selection (i.e., those chimerae that have a properly expressed FLAG tag are the desired in-frame chimerae and are selected). MRD_PR2 is a shorter primer containing the FLAG tag.

**Table 2:**

| SEQ ID NO: | Name | Sequence (5' -3') |
|---|---|---|
| SEQ ID NO: 1 | 1A | TGC CGG AGT CAG CGT |
| SEQ ID NO: 2 | 1B | AGT CAG AGT CGC CAC |
| SEQ ID NO: 3 | MRD LP | |
| SEQ ID NO: 4 | MRD_LP+1 | |
| SEQ ID NO: 5 | MRD_LP+2 | |
| SEQ ID NO: 6 | MRD RP1 | |
| SEQ ID NO: 7 | MRD RP2 | |

### Example 2

### Amplification

### Step 1: PCR

A reaction mixture was assembled containing template DNA, polymerase buffer, Hot-Start polymerase, forward and reverse primers, MgCl₂ and dNTPs.

Cycling conditions were as follows: Step 1: 15 minutes at 95°C; Step 2: 1minute at 95°C; Step 3: 1 minute at 58°; Step 4: 1 minute at 72°; Step 5: Go back to step 2 and repeat 17 times; Step 6: 10 minutes at 72°; Step 7: Finish and hold at 3.5°. As the primers incorporate the elements necessary for transcription, translation and purification, the amplification products of the *in situ* PCR are now ready to undergo the remaining steps of the process.

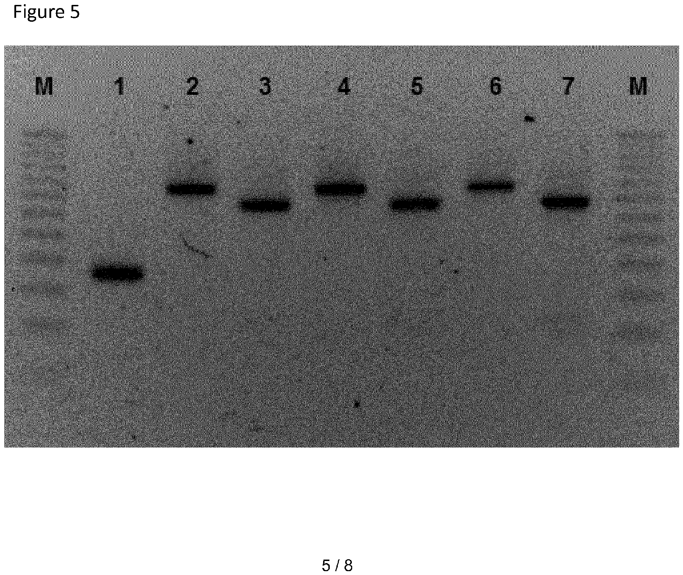

2uL were taken from the 25uL PCR reaction and run on a 4% agarose gel (Figure 5) In Figure 5, the M lane is the molecular weight ladder. The primers used to synthesize the products in each lane are shown below in Table 3:

**Table 3:**

| Lane | Left Primer | Right Primer |
|---|---|---|
| 1 | 1A | 1B |
| 2 | MRD_LP | MRD_RP1 |
| 3 | MRD_LP | MRD_RP2 |
| 4 | MRD_LP+1 | MRD_RP1 |
| 5 | MRD_LP+1 | MRD_RP2 |
| 6 | MRD_LP+2 | MRD_RP1 |
| 7 | MRD_LP+2 | MRD_RP2 |

Lane 1 shows the product formed when the primers contain only the target specific elements without the transcription/translational elements or the tags included (primers 1A and 1B). In lanes 2-3, the "in-frame" primer is used as left primer (MRD_LP), while the right primer is varied and has both FLAG and Strep tags (MRD_RP1, lane 2) and only FLAG tags (MRD_RP2, lane 3). In lanes 4-5, the left primer is the "N+1" out-of-frame primer (MRD_LP+1), while the right primer is varied to have both tags (MRD_RP1, lane 4) or only FLAG tag (MRD_RP2, lane 5). In lanes 6-7, the left primer is the "N+2" out-of-frame primer (MRD_LP+2), while the right primer is varied to have both tags (MRD_RP1, lane 6) or just FLAG tag (MRD_RP2, lane 7).

As can be seen from Figure 5, the in situ PCR produces the expected results. There is a lower molecular weight product in lane 1 when the primers do not contain the transcription/translation elements or the tag elements. In each of the other lanes, the lanes with the two-tag right primer formed slightly higher molecular weight products than the products formed with one-tag primers. All the bands are approximately the same intensity and appropriately sized.

### Step 2. mRNA library

In this step, DNA was converted to RNA using in-vitro transcription kit from Promega (T7 RiboMAX in-vitro transcription kit) according to manufacturer's instructions.

The following T7 RiboMax (Promega) transcription reagents (50uL) were assembled:

**Table 4:**

| Reagent | Amount (uL) |
|---|---|
| 5X T7 buffer | 10.00 |
| 25 mM rNTP each | 12.5 |
| -250 ng/ul DNA library | 22.5 |
| Enzyme mix | 5.0 |
| *Total* | *50* |

The mixture was incubated at 37°C for 3h. 8uL of RQ DNase (1U/uL) was added, and incubated at 37°C for 1 hour. Purification was performed with RNeasy mini (Qiagen), with elution in 26uL Qiagen RNeasy H₂O (yield is generally ∼1000 ng/uL).

### Step 3. Ligate puromycin spacer to mRNA.

Ligation of the puromycin linker (custom synthesis via Biosearch Technologies, Inc) to the transcribed RNA occurred via T4 RNA ligase, which allowed for the ligation of 2 single stranded RNA species. mRNA was heated at 75°C for 1 min (then snap chilled) before assembling the ligase reaction. The following reagents (50L) were assembled:

**Table 5:**

| Reagent | Amount (uL) |
|---|---|
| 10X T4 RNA ligase buffer | 5.0 |
| 10 mM ATP | 1.5 |
| mRNA library | 13.5 |
| 100 uM puromycin | 20.0 |
| T4 RNA ligase | 10.0 |
| *Total* | *50. 0* |

The mRNA was incubated at 15°C for 2h. Samples were purified using RNeasy column, eluted in 51uL Qiagen RNeasy H₂O (yield is generally -200 ng/uL).

### Step 4. PURExpress translation of mRNA-spacer.

The RNA-puromycin construct was translated into RNA-puromycin-peptide fusion using New England Biolab's PureExpress In-vitro translation kit as directed. As translation of the RNA-puromycin construct proceeds, ribosome moves along the RNA template, and once it reaches the 3' end of the template, the fused puromycin will enter the ribosome's A site and be incorporated into the nascent peptide. The mRNA-polypeptide fusion is then released from the ribosome, resulting the RNA-puromycin-peptide fusion or chimera. The following reagents (25uL) were assembled:

**Table 6:**

| Reagent | Amount (uL) |
|---|---|
| Solution A | 10.0 |
| Solution B (minus RF123) | 7.5 |
| Rnasin (RNase inhibitor) | 1.0 |
| mRNA-spacer | 6.5 |
| *Total* | 25 |

Incubation occurred at 37°C for 1h.

### Step 5. Purification

In-frame translated fusion products will correctly translate the affinity purification tag, in this case the FLAG tag, whereas any frameshift mistakes (i.e. N +/- 1, N +/-2) will not. Purification in this case was done via the short peptide FLAG tag (DYKDDDDK) and anti-FLAG antibody coated magnetic beads. All fusions with a correctly translated FLAG tag will bind to the anti-FLAG antibody that is conjugated to the magnetic bead, whereas frameshift mistakes will be washed away. Additionally, non-specific DNA is digested using Promega's RNase-free DNase RQ1. The beads with anti-FLAG antibody are commercially available from Sigma.

To perform the purification, the reaction was mixed with 200uL of TBSTE [TBST (10 mM TriszHCl, pH 8.0, 150 mM NaCl, 0.02% Tween-20) with 2 mM EDTA]. 20uL of anti-flag magnetic beads were added. This mixture was incubated at 4C for 1h on a rotating platform. The magnetic beads were washed 3X with 300uL TBSTE, then washed again 1X with 300uL Qiagen RNeasy H₂O. The resulting sample is then subjected to RQ1 treatment. RQ1 is a RNase-free DNase for removal of any subsisting DNA in the sample. To perform this, the sample was resuspended in 100uL of RQ1 buffer, 10uL of RQ1 was added, sample was incubated at 37°C for 1h. After incubation, the magnetic beads were washed 3X with 300uL TBSTE, then washed 1X with 300uL Qiagen RNeasy H₂O.

### Step 6: RT Reaction

RNA was converted back to cDNA via Invitrogen SuperScript reverse transcriptase. Elution of the cDNA from the magnetic beads was done using mild denaturant (in this case 0.1N NaOH). Magnetic beads were discarded. To perform this step, the following protocol was used. Resuspend the magnetic beads with the following reagents (80uL):

**Table 7:**

| Reagents | Amount (uL) |
|---|---|
| 5X RT buffer | 16.00 |
| Rnase free H2O | 46.00 |
| 10 mM dNTP | 4.00 |
| 2uM reverse primer* | 4.00 |
| | |
| RP1 or RP2 depending on the experiment | |
| 0.1 M DTT | 8.00 |
| RNasin | 2.00 |
| Superscript RT (Invitrogen) | 0.5 |
| *Total* | *80. 0* |

The above master mix was mixed with the beads and incubated at 37°C for 30 min. Beads were then washed 2X with 300uL TBSTE, and washed again 1X with 300uL Qiagen RNeasy water.

The fusion products were then eluted with 0.1N NaOH by the following protocol: 1) Elute in 100uL 0.1N NaOH; 2) Add 1ul of 100X tRNA as carrier; 3) Elute at room temperature for 3 minutes; 4) Purify in Millipore column, 5000 rpm 1 min; 5) Ethanol precipitate (NaAc + EtOH) the flow through; 6) Resuspend in 100uL H2O.

### Step 7. Amplification.

The cDNA was amplified using Phusion polymerase from NEB to reconstitute the purified construct from step 1. Assemble the following reagents (400uL):

**Table 8:**

| Reagents | Amount (uL) |
|---|---|
| 5X HF buffer | 80 |
| 10 uM forward primer* | 8 |
| 10 uM reverse primer* | 8 |
| 10 mM dNTP | 8 |
| Eluted fusions | 100 |
| H2O | 194 |
| Phusion polymerase | 2 |
| *Total* | *400* |

Stepwise PCR was run to assess amplification quality after different cycles. The following conditions were used: Step 1: 1 minute at 98°C; Step 2: 20 seconds at 95°C; Step 3: 1 minute at 64°C; Step 4: 30 seconds at 72°C; Step 5: Go back to step 2 and repeat x times (depending upon which step in the PCR is desired for evaluation); Step 6: 2 minute at 72°C; Step 7: hold at 3.5°C.

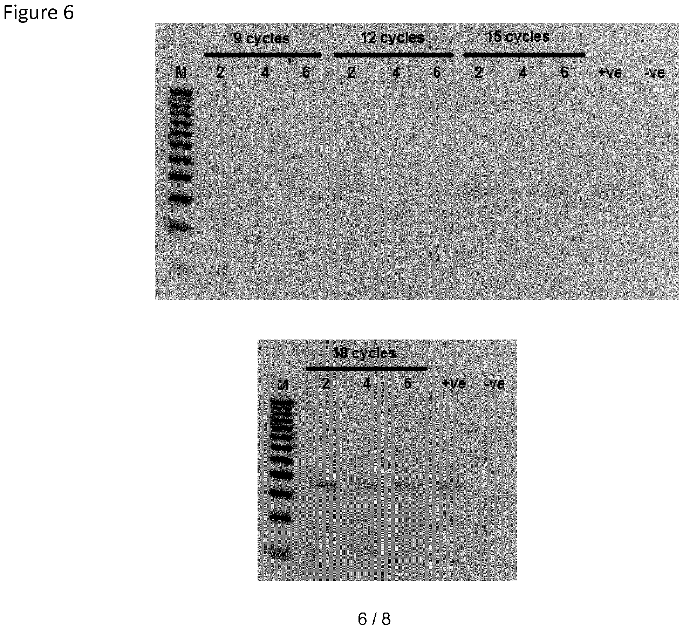

After in-vitro transcription/translation, purification and reverse transcription, a PCR cycle titration was performed. An electrophoresis gel separating products taken at various amplification steps is shown in Figure 6. The primers used to make the products in the various lanes are shown in Table 9:

**Table 9:**

| Lane | Left Primer | Right Primer |
|---|---|---|
| 2 | MRD_LP | MRD_RP2 |
| 4 | MRD_LP+1 | MRD_RP2 |
| 6 | MRD_LP+2 | MRD_RP2 |

It should be noted that these lanes in Figure 6 (lanes 2, 4, and 6) correspond to the products found in lanes 3, 5 and 7 of Figure 5. As such, these are all one-tag embodiments (FLAG tag), while the left primer used is varied from in-frame (N, lane 2), to out of frame (N+1, lane 4 and N+2, lane 6). Amplification products were evaluated after 9, 12, 15 and 18 cycles.

With sample 2, which represents the in-frame translation of the FLAG purification tag, PCR product is seen by cycle 12, whereas with sample 4/6, PCR product is seen by cycle 15, which represents approximately 8 fold enrichment. By 18 cycles, the difference between the in-frame and the out-of-frame products is readily visible.

### Example 3

### Sequencing the purified oligonucleotides

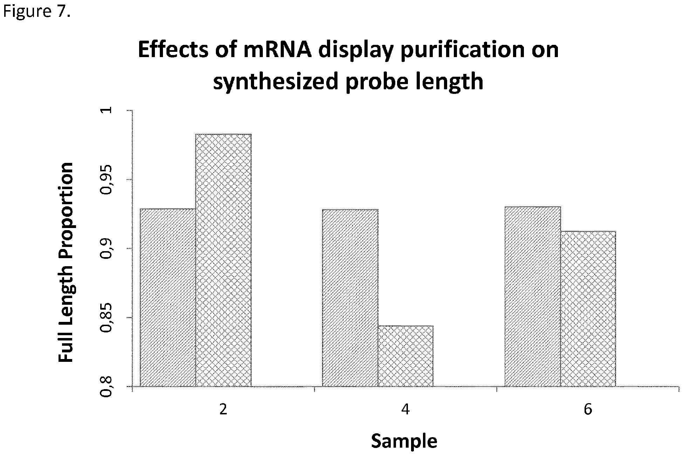

Libraries were sequenced using the MiSEQ instrument. Oligonucleotide was purchased and evaluated for purity (percentage of full length constructs) both before and after undergoing the inventive method. It is well known that purchased oligonucleotides will contain a certain percentage of imperfectly formed moieties. Figure 7 shows the effects of using the mRNA display-based purification on the purchased oligonucleotide. The first column of each pair represents the percentage of the purchased oligonucleotides that are full length prior to any purification. The second column of each pair represents the percentage of the oligonucleotides that are full length from lane 2 (in-frame), lane 4 (out-of-frame, N+1), and lane 6 (out-of-frame, N+2) as shown in Figure 6.

As can be seen from Figure 7, purification using FLAG tag and FLAG antibodies results in a positive increase in the full-length percentage as compared to the commercial product. The percentage of full-length oligonucleotides present increase from approximately 93% to approximately 98%. This difference in purity is important in certain applications that require high purity oligonucleotides.

It should be noted that the data used in Figure 7 show results from only 1 cycle of the inventive method. The purified oligonucleotides could be put through one or more additional cycles to increase the purity even further.

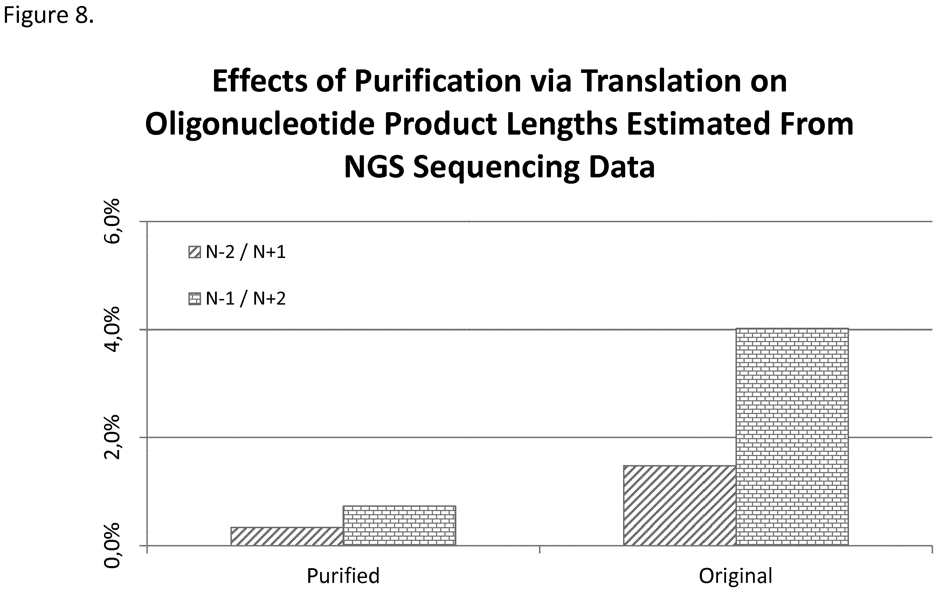

Figure 8 shows the amounts of the out-of-frame products found in the originally purchased product versus the amounts of such products in the purified sample. The original product contained approximately 5% N+1 out-of-phase product and approximately 1% N+2 out-of-phase product. After undergoing one cycle of the purification method of the present invention, the amounts of both out-of-phase products decreased substantially.

### Example 4

### Two tag system

In this example, steps 1-4 are as described in Example 2 are performed using the two-tag primer (e.g., the products shown in lanes 2, 4, and 6 in Figure 5). This procedure results in a chimeric pool having FLAG tag expressed in those moieties whose expression is in-frame, and Strep tag expressed in those moieties that are one step out of frame. However, in Step 5 there are two rounds of purification. The first step of purification involves incubating the samples with magnetic beads conjugated to anti-Strep antibodies. The magnetic beads are removed from the sample, thus removing the one-stepout-of-frame moieties (in this example, N-1 or N+2), which can then be discarded (negative selection). After the negative selection, the remaining sample can then proceed through steps 5-7 as described above, thereby selecting for the FLAG tagged moieties through use of anti-Flag antibodies conjugated to magnetic beads. Thus there is essentially a "pre-purification" step that removes the moieties that are one step out of frame from being further selected or amplified. The moieties that are two steps out of frame (in this example, N-2 or N+1) are negatively selected through the use of the anti-Flag antibodies.

### Example 5

### Three-tag system (prophetic)

In this example, steps 1-4 are carried out as described in Example 2; however, the second primer contains a nucleotide sequence that is expressed as a third peptide tag when the transcription/translation is out of frame by two nucleotides. The peptide sequence is not important other than that antibodies must be available (or synthesized) that are specific for the peptide sequence. For purposes of this example, the third tag is denominated as TAG3.

In an example of the three tag system, after step 4, FLAG tag is expressed only in in-frame products (N), Strep tag is expressed in only those moieties that are one step out of frame and TAG3 is expressed only in those moieties that are two steps out of frame. Instead of step 5 having two substeps, as in Example 4, it would now have 3 substeps wherein, for example, the first step is to incubate the sample with anti-TAG3 antibody beads and remove them from the remaining sample, the second substep is to incubate with anti-Step antibody beads and remove them from the sample, and the third substep is to incubate the remaining sample with anti-FLAG beads, collect those beads and elute the desired FLAG-tagged product. It is also possible to do both negative collections of the Strep and TAG3 products through simultaneous incubation with anti-Strep and anti-TAG3 beads, thus only having two substeps.

## Claims

1. A method of purifying a full-length target oligonucleotide from a pool further containing shorter or longer same-sequence oligonucleotide by-products, the method comprising:
a) amplifying the pool to form a second pool of oligonucleotides;
b) transcribing the second pool of oligonucleotides to form an RNA pool wherein the RNA molecules contain codons for at least one tag sequence;
c) ligating the molecules of the RNA pool to puromycin;
d) translating the puromycin-ligated RNA molecules to form a pool of chimeric molecules containing the RNA linked to a peptide expressed from the RNA;
e) capturing and isolating the chimeric molecules that express the tag;
f) reverse transcribing the RNA of the isolated chimeric moieties to form a pool of cDNA comprising the purified full-length oligonucleotide.

2. The method of claim 1, wherein amplification in step a) uses at least two primers comprising a target binding site, and further comprising one or more of a promoter, an enhancer, a ribosome binding site, a translation initiation site and a sequence encoding at least one tag.

3. The method of claim 2, wherein the first primer comprises promoter, enhancer, the ribosome binding site and the translation initiation site and the second primer comprises the sequence encoding at least one tag.

4. The method of claim 2, wherein amplification in step a) uses the second primer comprising the sequence encoding a first tag and a sequence encoding a second tag.

5. The method of claim 1, wherein codons of the first tag sequence are in frame with the full-length oligonucleotide and out of frame with shorter and with longer same-sequence oligonucleotide by-products.

6. The method of claim 1, wherein capturing in step e) is performed with a tag-specific binding agent.

7. The method of claim 6, wherein the tag-specific binding agent is an antibody.

8. The method of claim 5, wherein capturing in step e) comprises capturing of the first tag sequence.

9. The method of claim 1, further comprising amplifying the pool of cDNA comprising the purified full-length oligonucleotide.

10. The method of claim 1, further comprising repeating one or more cycles of steps b)-f) using the amplified pool of cDNA as the second pool of oligonucleotides.

11. A kit for purifying a full-length target oligonucleotide from a pool further containing shorter or longer same-sequence oligonucleotide by-products, the kit comprising a pair of primers wherein the first primer comprises a target binding site, a promoter, an enhancer, a ribosome binding site and a translation initiation site and the second primer comprises the sequence encoding at least one tag, further comprising the following: reagents for DNA amplification, reagents for DNA transcription, reagents for nucleic acid ligation, puromycin, reagents for RNA translation, at least one tag-binding agent specific for the at least one tag and reagents for RNA reverse transcription.

12. The kit of claim 11, wherein the second primer comprises the sequence encoding a first tag; and the further a sequence encoding a second tag.

## Patentansprüche

1. Verfahren zur Reinigung eines vollständigen Ziel-Oligonukleotids aus einem Pool, der ferner kürzere oder längere Oligonukleotid-Nebenprodukte der gleichen Sequenz enthält, wobei das Verfahren umfasst:
a) Amplifizieren des Pools, um einen zweiten Pool von Oligonukleotiden zu bilden;
b) Transkribieren des zweiten Pools von Oligonukleotiden, um einen RNA-Pool zu bilden, wobei die RNA-Moleküle Codone für mindestens eine Tag-Sequenz enthalten;
c) Ligieren der Moleküle des RNA-Pools an Puromycin;
d) Translatieren der Puromycin-ligierten RNA-Moleküle, um einen Pool von chimären Molekülen zu bilden, die die RNA enthalten, die mit einem von der RNA exprimierten Peptid verknüpft ist;
e) Einfangen und Isolieren der chimären Moleküle, die das Tag exprimieren;
f) reverses Transkribieren der RNA der isolierten chimären Teile, um einen Pool von cDNA zu bilden, der das gereinigte vollständige Oligonukleotid umfasst.

2. Verfahren nach Anspruch 1, wobei die Amplifikation in Schritt a) mindestens zwei Primer verwendet, die eine Zielbindungsstelle umfassen und ferner eine/n oder mehrere von einem Promotor, einem Verstärker, einer Ribosomenbindungsstelle, einer Translationsinitiierungsstelle und einer Sequenz, die mindestens ein Tag kodiert, umfassen.

3. Verfahren nach Anspruch 2, wobei der erste Primer Promotor, Verstärker, die Ribosomenbindungsstelle und die Translationsinitiierungsstelle umfasst und der zweite Primer die Sequenz, die mindestens ein Tag kodiert, umfasst.

4. Verfahren nach Anspruch 2, wobei die Amplifikation in Schritt a) den zweiten Primer verwendet, der die Sequenz, die ein erstes Tag kodiert, und eine Sequenz, die ein zweites Tag kodiert, umfasst.

5. Verfahren nach Anspruch 1, wobei sich Codone der ersten Tag-Sequenz im Raster mit dem vollständigen Oligonukleotid und außerhalb des Rasters mit kürzeren und mit längeren Oligonukleotid-Nebenprodukten der gleichen Sequenz befinden.

6. Verfahren nach Anspruch 1, wobei das Einfangen in Schritt e) mit einem Tag-spezifischen Bindemittel durchgeführt wird.

7. Verfahren nach Anspruch 6, wobei das Tag-spezifische Bindemittel ein Antikörper ist.

8. Verfahren nach Anspruch 5, wobei das Einfangen in Schritt e) das Einfangen der ersten Tag-Sequenz umfasst.

9. Verfahren nach Anspruch 1, das ferner das Amplifizieren des Pools von cDNA umfasst, der das gereinigte vollständige Oligonukleotid umfasst.

10. Verfahren nach Anspruch 1, das ferner das Wiederholen eines oder mehrerer Zyklen der Schritte b)-f) unter Verwendung des amplifizierten Pools von cDNA als den zweiten Pool von Oligonukleotiden umfasst.

11. Kit zur Reinigung eines vollständigen Ziel-Oligonukleotids aus einem Pool, der ferner kürzere oder längere Oligonukleotid-Nebenprodukte der gleichen Sequenz enthält, wobei das Kit ein Paar von Primern umfasst, wobei der erste Primer eine Zielbindungsstelle, einen Promotor, einen Verstärker, eine Ribosomenbindungsstelle und eine Translationsinitiierungsstelle umfasst und der zweite Primer die Sequenz umfasst, die mindestens ein Tag kodiert, und ferner Folgendes umfasst: Reagenzien für die DNA-Amplifikation, Reagenzien für die DNA-Transkription, Reagenzien für die Nukleinsäure-Ligation, Puromycin, Reagenzien für die RNA-Translation, mindestens ein für das mindestens eine Tag spezifisches Tag-Bindemittel und Reagenzien für die reverse RNA-Transkription.

12. Kit nach Anspruch 11, wobei der zweite Primer die Sequenz, die ein erstes Tag kodiert, und ferner eine Sequenz, die ein zweites Tag kodiert, umfasst.

## Revendications

1. Méthode de purification d'un oligonucléotide cible de pleine longueur à partir d'un pool contenant des sous-produits plus courts ou plus longs ayant la même séquence que l'oligonucléotide, la méthode comprenant :
a) l'amplification du pool afin de former un second pool d'oligonucléotides ;
b) la transcription du second pool d'oligonucléotides afin de former un pool d'ARN dans lequel les molécules d'ARN contiennent des codons pour au moins une séquence de marquage ;
c) la ligature des molécules du pool d'ARN à la puromycine ;
d) la translation des molécules d'ARN ligaturées à la puromycine afin de former un pool de molécules chimériques contenant l'ARN liés à un peptide exprimé à partir de l'ARN ;
e) la capture et l'isolement des molécules chimériques qui expriment le marqueur ;
f) la transcription inverse de l'ARN des fragments chimériques isolés afin de former un pool d'ADNc comprenant l'oligonucléotide de pleine longueur purifié.

2. Méthode selon la revendication 1, dans laquelle l'amplification de l'étape a) utilise au moins deux amorces comprenant un site de liaison cible, et comprenant en outre un ou plusieurs éléments choisis parmi un promoteur, un activateur, un site de liaison ribosomique, un site initiateur de translation et une séquence codant pour au moins un marqueur.

3. Méthode selon la revendication 2, dans laquelle la première amorce comprend un promoteur, un activateur, le site de liaison ribosomique et le site initiateur de traduction et la seconde amorce comprend la séquence codant pour au moins un marqueur.

4. Méthode selon la revendication 2, dans laquelle l'amplification de l'étape a) utilise la seconde amorce comprenant la séquence codant pour un premier marqueur et une séquence codant pour un second marqueur.

5. Méthode selon la revendication 1, dans laquelle les codons de la première séquence de marquage sont dans le cadre avec l'oligonucléotide de pleine longueur et hors cadre avec les sous-produits plus courts et plus longs ayant la même séquence que l'oligonucléotide.

6. Méthode selon la revendication 1, dans laquelle la capture de l'étape e) est effectuée avec un agent se liant spécifiquement à un marqueur.

7. Méthode selon la revendication 6, dans laquelle l'agent se liant spécifiquement à un marqueur est un anticorps.

8. Méthode selon la revendication 5, dans laquelle la capture de l'étape e) comprend la capture de la première séquence de marquage.

9. Méthode selon la revendication 1, comprenant en outre une amplification du pool d'ADNc comprenant l'oligonucléotide de pleine longueur purifié.

10. Méthode selon la revendication 1, comprenant en outre la répétition d'un ou plusieurs cycles des étapes b) à f) utilisant le pool d'ADNc amplifié comme second pool d'oligonucléotides.

11. Kit de purification d'un oligonucléotide cible de pleine longueur à partir d'un pool contenant en outre les sous-produits plus courts ou plus longs ayant la même séquence que l'oligonucléotide, le kit comprenant une paire d'amorces, la première amorce comprend un site de liaison cible, un promoteur, un activateur, un site de liaison ribosomique et un site initiateur de traduction, et la seconde amorce comprenant la séquence codant pour au moins un marqueur comprenant en outre les éléments suivants : réactifs pour l'amplification de l'ADN, réactifs pour la transcription de l'ADN, réactifs pour la ligature de l'acide nucléique, puromycine, réactifs pour la traduction de l'ARN, au moins un agent se liant un marqueur spécifique pour au moins un marqueur, et réactifs pour la transcription inverse de l'ARN.

12. Kit de revendication 11, dans lequel la seconde amorce comprend la séquence codant pour un premier marqueur ; et en outre une séquence codant pour un second marqueur.
